# EUROPÄISCHE PATENTSCHRIFT

(11) **EP 4 370 080 B1**
(45) Veröffentlichungstag und Bekanntmachung des Hinweises auf die Patenterteilung: **20.11.2024**
(21) Anmeldenummer: 22754015.0
(22) Anmeldetag: 15.07.2022
(51) Int. Cl.: A61F 9/007, A61M 1/00, A61M 3/02

(54) **OPHTHALMOCHIRURGISCHE EINRICHTUNG**
OPHTHALMIC SURGICAL INSTRUMENT
INSTRUMENT CHIRURGICAL OPHTALMIQUE

(30) Priorität: 21.09.2021 DE 102021210483
(43) Veröffentlichungstag der Anmeldung: 22.05.2024
(73) Patentinhaber: Carl Zeiss Meditec AG, 07745 Jena (DE)
(72) Erfinder: KUEBLER, Christoph, 73447 Oberkochen (DE); MADLINGER, Philipp, 73433 Aalen (DE); KOHLHAMMER, Susanne, 89134 Blaustein (DE)
(74) Vertreter: Carl Zeiss AG - Patentabteilung
(86) Internationale Anmeldenummer: PCT/EP2022/069879
(87) Internationale Veröffentlichungsnummer: WO 2023/046331

(56) Entgegenhaltungen:
- DE-A1- 102012 018 983
- DE-A1- 102015 003 799
- DE-B3- 102016 201 297
- US-A1- 2019 060 533

## Beschreibung

Die Erfindung betrifft eine ophthalmochirurgische Einrichtung.

Zur Behandlung einer Augenlinsentrübung, welcher in der Medizin als grauer Star bezeichnet wird, gibt es mehrere chirurgische Techniken. Die am weitesten verbreitete Technik ist die Phakoemulsifikation, bei der eine dünne Hohlnadel in die Augenlinse eingeführt und zu Ultraschallschwingungen angeregt wird. Die vibrierende Hohlnadel zerkleinert in ihrer nächsten Umgebung die Linse derart, dass viele kleine Linsenpartikel entstehen, welche durch eine Leitung mittels einer Pumpe abgesaugt werden können. Dabei wird ein Irrigationsfluid (Spülfluid) mittels einer Irrigationsfluidleitung zugeführt, wobei das Absaugen der Linsenpartikel mit dem Fluid, welches zusammen als Aspirationsfluid bezeichnet wird, durch eine Aspirationsfluidleitung erfolgt. Ist die Linse vollständig emulsifiziert und entfernt worden, kann in den leeren Kapselsack eine neue künstliche Linse eingesetzt werden, sodass ein derart behandelter Patient wieder ein gutes Sehvermögen erreichen kann.

Das Zerkleinern der Linse durch eine mit Ultraschall schwingende Hohlnadel ist bewährt und funktioniert gut. Ein prinzipielles Problem besteht aber darin, dass die Größe der zerkleinerten Partikel unterschiedlich ausfällt und solche Partikel die Hohlnadel an ihrem Eingangsbereich verstopfen können. Das Absaugen des Aspirationsfluids gelingt dann nur noch schwer oder gar nicht mehr. Dieser Zustand wird als Okklusion bezeichnet. In der Aspirationsfluidleitung wird dann sämtliches noch vorhandene Fluid abgesaugt, sodass sich bei entsprechend lang anhaltender Okklusion ein hoher Unterdruck aufbauen kann. Ein gefährlicher Zustand entsteht dann, wenn sich das Partikel von der Hohlnadel wieder löst und Aspirationsfluid wieder in die Hohlnadel und die Aspirationsfluidleitung einströmen kann. Aufgrund des Unterdruckes in der Aspirationsfluidleitung entsteht ein sprungartiger Druckausgleich, der im Auge zu einem Kollaps und zu gefährlichen Komplikationen für einen Patienten führen kann. In DE 10 2019 216 670 A1 ist eine relativ komplizierte ophthalmochirurgische Steuerungsmodulvorrichtung beschrieben.

Es besteht daher eine Aufgabe, eine ophthalmochirurgische Einrichtung zu schaffen, mit welcher eine Okklusion und das Aufbrechen einer Okklusion schnell und zuverlässig erkannt werden können.

Die Aufgabe wird durch den Gegenstand des unabhängigen Patentanspruchs gelöst. Vorteilhafte Weiterbildungen der Erfindung sind Gegenstand der Unteransprüche.

Die erfindungsgemäße ophthalmochirurgische Einrichtung, weist auf:
- Eine Irrigationsfluidleitung, durch welche Irrigationsfluid von einem Irrigationsfluidbehälter zu einem ophthalmochirurgischen Handstück strömen kann,
- eine Aspirationsfluidleitung, durch welche Aspirationsfluid von dem ophthalmochirurgischen Handstück zu einem Sammelbehälter strömen kann,
- eine erste Volumenstrom-Bestimmungsvorrichtung, welche in Strömungsrichtung vor dem Handstück angeordnet ist und eingerichtet ist, einen ersten Volumenstrom in der Irrigationsfluidleitung zu bestimmen,
- eine zweite Volumenstrom-Bestimmungsvorrichtung, welche in Strömungsrichtung nach dem Handstück angeordnet ist und eingerichtet ist, einen zweiten Volumenstrom in der Aspirationsfluidleitung zu bestimmen,
- eine Okklusions-Bestimmungsvorrichtung, welche mit der ersten Volumenstrom-Bestimmungsvorrichtung und der zweiten Volumenstrom-Bestimmungsvorrichtung gekoppelt ist und eingerichtet ist, den zeitlichen Verlauf des ersten Volumenstroms und den zeitlichen Verlauf des zweiten Volumenstroms zu erfassen, jeweils einen Vergleich mit gespeicherten zeitlichen Verläufen eines ersten Volumenstroms in der Irrigationsfluidleitung bei unterschiedlichen Okklusionszuständen und gespeicherten zeitlichen Verläufen eines zweiten Volumenstroms in der Aspirationsfluidleitung bei unterschiedlichen Okklusionszuständen vorzunehmen und auf Basis der Vergleiche einen momentanen Okklusionszustand in der Aspirationsfluidleitung zu bestimmen.

Um eine Information über eine Okklusion zu erhalten, haben die Erfinder beobachtet, dass die Kenntnis eines Volumenstroms vor dem Handstück und nach dem Handstück sehr aussagekräftig ist. Vorteilhaft ist dabei, dass ein Volumenstrom sehr schnell, sehr genau und mit geringer Unsicherheit ermittelt werden kann. Die Erfinder haben zudem beobachtet, dass nicht nur der Volumenstrom in seiner absoluten Höhe, sondern auch in seinem zeitlichen Verlauf einen Rückschluss auf die vorhandene Intensität oder einen Zustand einer Okklusion oder eines Aufbrechens der Okklusion ermöglicht. Es gibt typische Verläufe für einen Volumenstrom bei keiner Okklusion, bei einer teilweisen Okklusion, bei vollständiger Okklusion oder beliebigen Situationen, die sich zwischen diesen Zuständen einteilen lassen. Derartige zeitliche Verläufe eines ersten Volumenstroms und eines zweiten Volumenstroms können in einem Speicher gespeichert werden. Gemäß der Erfindung wird der zeitliche Verlauf eines ersten Volumenstroms in der Irrigationsfluidleitung erfasst und mit zuvor gespeicherten zeitlichen Verläufen eines ersten Volumenstroms in der Irrigationsfluidleitung, die bei unterschiedlichen Okklusionszuständen ermittelt wurden, verglichen. Dies erfolgt in gleicher Weise mit dem zweiten Volumenstrom. Gemäß der Erfindung wird der zeitliche Verlauf eines zweiten Volumenstroms in der Aspirationsfluidleitung erfasst und mit zuvor gespeicherten zeitlichen Verläufen eines zweiten Volumenstroms in der Aspirationsfluidleitung, die bei unterschiedlichen Okklusionszuständen ermittelt wurden, verglichen. Auf Basis dieser Vergleiche kann dann ein momentaner Okklusionszustand in der Aspirationsfluidleitung bestimmt werden.

Das Erstellen einer Korrelation zwischen dem zeitlichen Verlauf eines erfassten Volumenstroms und einem zeitlichen Verlauf eines gespeicherten Volumenstroms durch eine OkklusionsBestimmungsvorrichtung erfordert nur geringe Rechenleistung, sodass dies sehr schnell erfolgen kann. Es hat sich herausgestellt, dass eine solche Korrelation auch sehr genau und mit geringer Unsicherheit gelingt. Mit der ophthalmochirurgischen Einrichtung ist es damit möglich, schnell und zuverlässig zu erkennen, ob überhaupt eine Okklusion vorliegt, in welcher Intensität diese Okklusion vorliegt und ob eine Okklusion gerade aufbricht. Es ist also mit der ophthalmochirurgischen Einrichtung möglich, einen momentanen Okklusionszustand in der Aspirationsfluidleitung schnell und zuverlässig zu bestimmen.

Es wird darauf hingewiesen, dass die Okklusions-Bestimmungsvorrichtung sowohl den zeitlichen Verlauf des ersten Volumenstroms in der Irrigationsfluidleitung als auch den zeitlichen Verlauf des zweiten Volumenstroms in der Aspirationsfluidleitung erfasst und einen Vergleich mit gespeicherten zeitlichen Verläufen sowohl eines ersten Volumenstroms in der Irrigationsfluidleitung als auch mit gespeicherten zeitlichen Verläufen eines zweiten Volumenstroms in der Aspirationsfluidleitung vornimmt. Ein Vergleich nur eines momentan erfassten zeitlichen Verlaufs eines zweiten Volumenstroms in der Aspirationsfluidleitung mit einem gespeicherten zeitlichen Verlauf eines zweiten Volumenstroms in der Aspirationsfluidleitung genügt nicht, um einen Okklusionszustand zu ermitteln.

Wenn nur eine solcher Vergleich für eine Situation in der Aspirationsfluidleitung vorgenommen wird, kann nicht erkannt werden, ob der zeitliche Verlauf des Volumenstroms in der Aspirationsfluidleitung und der zeitliche Verlauf des Volumenstroms in der Irrigationsfluidleitung symmetrisch zueinander verlaufen. Wenn keine Symmetrie der zeitlichen Verläufe in der Aspirationsfluidleitung und der Irrigationsfluidleitung erkennbar ist, kann dies ein Hinweis darauf sein, dass keine Okklusion, sondern ein anderes Problem vorliegt. Es kann sich dabei um folgende Probleme handeln, wobei diese Aufzählung nicht vollständig ist:
- Ein Schlauch der Irrigationsfluidleitung oder der Aspirationsfluidleitung ist abgeknickt, sodass keine Strömung vorliegt.
- Es gibt keine ausreichend hohe Strömung in der Irrigationsfluidleitung, da ein Leck vorliegt.
- Der Irrigationsfluidbehälter ist nahezu entleert.
- In der Irrigationsfluidleitung ist aus unbekannten Gründen ein Linsenpartikel enthalten und führt zu einer unüblichen Schwankung des Volumenstroms.
- Das zu behandelnde Auge hat eine schwere Verletzung.
- Die Einstichgröße für eine Hohlnadel in der Hornhaut ist zu klein, sodass zu wenig Irrigationsfluid in die Vorderkammer gelangen kann.

Ein Vergleich zwischen dem momentan erfassten zeitlichen Verlauf des ersten Volumenstroms in der Irrigationsfluidleitung mit einem gespeicherten zeitlichen Verlauf des ersten Volumenstroms in der Irrigationsfluidleitung und ein Vergleich zwischen dem momentan erfassten zeitlichen Verlauf des zweiten Volumenstroms in der Aspirationsfluidleitung mit einem gespeicherten zeitlichen Verlauf des zweiten Volumenstroms in der Aspirationsfluidleitung beinhaltet auch eine Überprüfung der Symmetrie des zeitlichen Verlaufs des momentan erfassten ersten Volumenstroms in der Irrigationsfluidleitung mit dem momentan erfassten zeitlichen Verlauf des zweiten Volumenstroms in der Aspirationsfluidleitung.

Gemäß einer Weiterbildung weist die ophthalmochirurgische Einrichtung eine Steuerungseinrichtung auf, welche eingerichtet ist, an einem Eingang von der OkklusionsBestimmungsvorrichtung ein Okklusionszustands-Signal zu empfangen und an einem Ausgang ein Steuersignal für mindestens einen Betriebsparameter des ophthalmochirurgischen Handstücks an das ophthalmochirurgische Handstück abzugeben.

Eine Okklusion tritt meistens ganz am Anfang der Aspirationsfluidleitung, also noch im ophthalmochirurgischen Handstück oder an der Spitze der Hohlnadel, auf. Es ist daher vorteilhaft, wenn bei Kenntnis eines Okklusionszustandes ein Betriebsparameter eines ophthalmochirurgischen Handstücks so gesteuert werden kann, dass zum Beispiel die Okklusion aufbrechen kann.

Der Betriebsparameter des ophthalmochirurgischen Handstücks kann eine elektrische Antriebsenergie sein. Die elektrische Antriebsenergie kann für eine longitudinale Bewegung oder transversale Bewegung oder Torsions-Bewegung einer Piezokeramik eines ophthalmochirurgischen Handstückes zur Phakoemulsifikation vorgesehen sein. Die Antriebsenergie ermöglicht es, ein Partikel, das zum Beispiel an der Spitze einer Hohlnadel den Eingang zur Hohlnadel verstopft, durch entsprechende Bewegung der Hohlnadel zu zerkleinern. Die Bewegung kann longitudinal erfolgen, also entlang einer Mittelachse der Hohlnadel, oder transversal erfolgen, also quer zu Mittelachse der Hohlnadel, oder durch eine Torsions-Bewegung erfolgen, also in einer Torsionsbewegung um die Mittelachse der Hohlnadel. Die Antriebsenergie kann zum Beispiel dazu genutzt werden, die Hohlnadel mit einem größeren Hub oder Weg anzutreiben und dadurch bei gleichbleibender Frequenz mehr Energie für das Zerkleinern des Partikels bereitzustellen.

Die elektrische Antriebsenergie kann auch für eine longitudinale Bewegung oder transversale Bewegung oder Torsions-Bewegung oder rotatorische Bewegung eines Schneidelementes eines Vitrektoms vorgesehen sein. Ein Vitrektom dient zum Schneiden zum Beispiel eines Glaskörpers und kann in vergleichbarer Weise wie ein Phakoemulsifikations-Handstück im Bereich der Schneidspitze durch zu große Partikel verstopfen. Wird mit der ophthalmochirurgischen Einrichtung erkannt, dass eine Okklusion beginnt, kann die Antriebsenergie dazu genutzt werden, das Schneidelement mit höherer Frequenz oder Schneidrate anzutreiben, bis wieder ein normaler Volumenstrom erreicht ist.

Gemäß einer Weiterbildung der Erfindung weist die ophthalmochirurgische Einrichtung auf:
- eine erste Fluidpumpe, welche in Strömungsrichtung zwischen dem Irrigationsfluidbehälter und der ersten Volumenstrom-Bestimmungsvorrichtung angeordnet ist und eingerichtet ist, Irrigationsfluid zum Handstück zu fördern,
- eine zweite Fluidpumpe, welche in Strömungsrichtung zwischen der zweiten Volumenstrom-Bestimmungsvorrichtung und dem Sammelbehälter angeordnet ist und eingerichtet ist, Aspirationsfluid zum Sammelbehälter zu fördern,
- ein erstes Zeitglied, welches eingerichtet ist, an seinem Eingang das Okklusionszustands-Signal zu empfangen, in Abhängigkeit von dem Okklusionszustands-Signal einen ersten Irrigationsfluid-Solldruck in Abhängigkeit von der Zeit zu ermitteln und an seinem Ausgang ein Signal für den ersten Irrigationsfluid-Solldruck der ersten Fluidpumpe abzugeben,
- ein zweites Zeitglied, welches eingerichtet ist, an seinem Eingang das Okklusionszustands-Signal zu empfangen, in Abhängigkeit von dem Okklusionszustands-Signal einen ersten Aspirationsfluid-Solldruck in Abhängigkeit von der Zeit zu ermitteln und an seinem Ausgang ein Signal für den ersten Aspirationsfluid-Solldruck der zweiten Fluidpumpe abzugeben.

Damit wird erreicht, dass mittels einer ersten Fluidpumpe der Druck in der Irrigationsfluidleitung und mittels einer zweiten Fluidpumpe der Druck in der Aspirationsfluidleitung geändert werden kann. Wird zum Beispiel erkannt, dass ein Partikel an der Spitze der Hohlnadel hängt und eine vollständige Okklusion in der Aspirationsfluidleitung bewirkt, kann die erste Fluidpumpe so gesteuert werden, dass der Druck in der Irrigationsfluidleitung abnimmt. Gleichzeitig kann die zweite Fluidpumpe so gesteuert werden, dass der Unterdruck in der Aspirationsfluidleitung sehr stark zunimmt. Damit kann versucht werden, das Partikel noch stärker an die Spitze der Hohlnadel zu ziehen und dadurch eventuell aufzubrechen. Zusätzlich kann auch die elektrische Antriebsenergie für das Handstück erhöht werden, sodass die Hohlnadel mit einem größeren Hub ausgelenkt wird und das Partikel zerbricht.

Die ophthalmochirurgische Einrichtung kann zusätzlich aufweisen:
- eine erste Auswertevorrichtung, welche eingerichtet ist, an einem ersten Eingang das Signal für den ersten Irrigationsfluid-Solldruck zu empfangen, und an einem zweiten Eingang ein Signal für einen zweiten Irrigationsfluid-Solldruck zu empfangen, und an einem Ausgang ein Signal für einen Irrigationsfluid-Steuerdruck der ersten Fluidpumpe zuzuführen, und
- eine zweite Auswertevorrichtung, welche eingerichtet ist, an einem ersten Eingang das Signal für den ersten Aspirationsfluid-Solldruck zu empfangen, und an einem zweiten Eingang ein Signal für einen zweiten Aspirationsfluid-Solldruck zu empfangen, und an einem Ausgang ein Signal für einen Aspirationsfluid-Steuerdruck der zweiten Fluidpumpe zuzuführen.

Der zweite Irrigationsfluid-Solldruck kann von einem dritten Zeitglied und der zweite Aspirationsfluid-Solldruck kann von einem vierten Zeitglied bereitgestellt werden, welche mit einem Fußpedal gekoppelt sind. Ein Bediener kann durch entsprechende Position des Fußpedals eine Größe für das dritte Zeitglied bereitstellen, sodass die erste Auswertevorrichtung mittels des zweiten Irrigationsfluid-Solldrucks und des ersten Irrigationsfluid-Solldrucks einen Irrigationsfluid-Steuerdruck ermittelt, welcher der ersten Fluidpumpe zugeführt werden kann. Dies kann analog dazu für die Aspirationsfluidleitung erfolgen. Durch entsprechende Position des Fußpedals kann der Bediener eine Größe für das vierte Zeitglied bereitstellen, sodass die zweite Auswertevorrichtung mittels des zweiten Aspirationsfluid-Solldrucks und des ersten Aspirationsfluid-Solldrucks einen Aspirationsfluid-Steuerdruck ermittelt, welcher der zweiten Fluidpumpe zugeführt werden kann. Dadurch kann zum Beispiel ein vorsichtiges Emulsifizieren der Augenlinse ermöglicht werden.

Weitere Vorteile und Merkmale der Erfindung werden mit Bezug auf die nachfolgenden Zeichnungen erklärt, in welchen zeigen:
- Fig. 1: eine schematische Darstellung einer Ausführungsform einer ophthalmochirurgischen Einrichtung, und
- Fig. 2: schematische Diagramme von Druckverläufen und Volumenstromverläufen und Energiezufuhr in Abhängigkeit von der Zeit bei einem Einsatz der ophthalmochirurgischen Einrichtung.

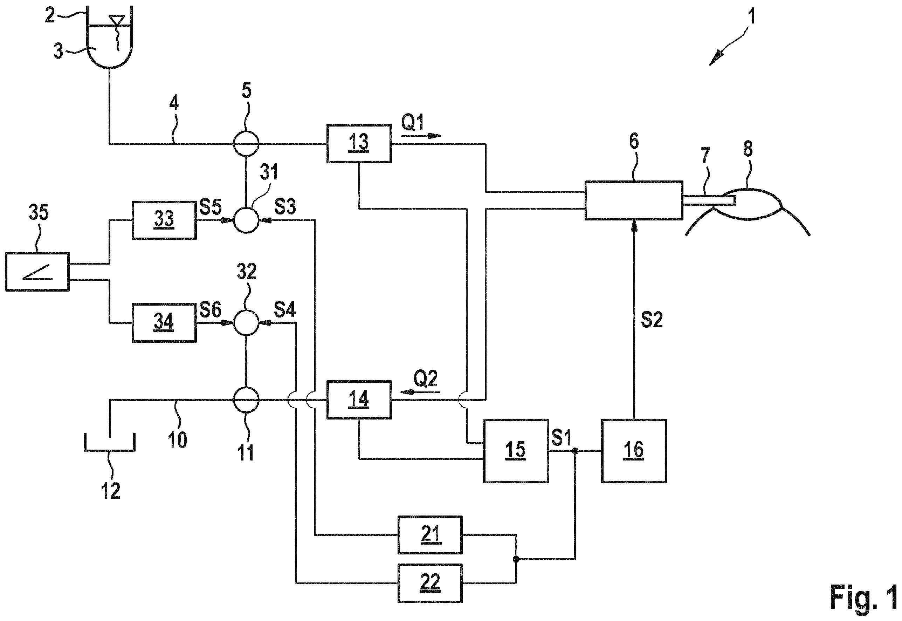

Figur 1 zeigt eine schematische Darstellung einer Ausführungsform einer ophthalmochirurgischen Einrichtung 1. In einem Irrigationsfluidbehälter 2 ist ein Irrigationsfluid 3 enthalten, welches durch eine daran angeschlossene Irrigationsfluidleitung 4 bis zu einem ophthalmochirurgischen Handstück 6 strömen kann. Dies kann durch eine erste Fluidpumpe 5 unterstützt werden, welche in Strömungsrichtung zwischen dem Irrigationsfluidbehälter 2 und dem Handstück 6 vorgesehen ist. Das Handstück 6 kann für die Phakoemulsifikation vorgesehen sein und besitzt eine Hohlnadel 7, welche sich in ein zu behandelndes Auge 8 einführen lässt. Zerkleinerte Partikel und Flüssigkeit können aus dem Auge 8 entlang einer Aspirationsfluidleitung 10 in Richtung zu einem Sammelbehälter 12 strömen. Diese Fluidbewegung wird durch eine zweite Fluidpumpe 11 veranlasst, welche in der Aspirationsfluidleitung 10 einen Unterdruck erzeugt.

In Strömungsrichtung zwischen der ersten Fluidpumpe 5 und dem Handstück 6 ist eine erste Volumenstrom-Bestimmungsvorrichtung 13 angeordnet, mit welcher ein erster Volumenstrom Q1 in der Irrigationsfluidleitung 4 bestimmt werden kann. Bevorzugt wird der erste Volumenstrom Q1 durch die Volumenstrom-Bestimmungsvorrichtung 13 indirekt bestimmt, indem zum Beispiel eine Position einer Membran oder eines Schwimmers erfasst und daraus der Volumenstrom ermittelt wird. In Strömungsrichtung zwischen dem Handstück 6 und der zweiten Fluidpumpe 11 ist eine zweite Volumenstrom-Bestimmungsvorrichtung 14 angeordnet, mit welcher ein zweiter Volumenstrom Q2 in der Aspirationsleitung 10 bestimmt werden kann. Auch der zweite Volumenstrom wird bevorzugt indirekt bestimmt. Die erste Volumenstrom-Bestimmungsvorrichtung 13 und die zweite Volumenstrom-Bestimmungsvorrichtung 14 sind mit einer Okklusions-Bestimmungsvorrichtung 15 gekoppelt, indem das Signal der ersten Volumenstrom-Bestimmungsvorrichtung 13 für den ersten Volumenstrom Q1 und das Signal der zweiten Volumenstrom-Bestimmungsvorrichtung 14 für den zweiten Volumenstrom Q2 zu der Okklusions-Bestimmungsvorrichtung 15 geleitet werden.

Die Okklusions-Bestimmungsvorrichtung 15 ist eingerichtet, den zeitlichen Verlauf des ersten Volumenstroms Q1 und den zeitlichen Verlauf des zweiten Volumenstroms Q2 zu erfassen. Die Okklusions-Bestimmungsvorrichtung 15 weist ein Speicherelement auf, in dem zeitliche Verläufe eines ersten Volumenstroms in der Irrigationsleitung und zeitliche Verläufe eines zweiten Volumenstroms in der Aspirationsleitung gespeichert sind. Jedem dieser gespeicherten zeitlichen Verläufe eines ersten Volumenstroms und eines zweiten Volumenstroms ist ein Okklusionszustand zugeordnet. Mittels der Okklusions-Bestimmungsvorrichtung 15 wird ein Vergleich zwischen einerseits dem zeitlichen Verlauf des ersten Volumenstroms, der mit der ersten Volumenstrom-Bestimmungsvorrichtung 13 erfasst wird, und dem zeitlichen Verlauf des zweiten Volumenstroms, der mit der zweiten Volumenstrom-Bestimmungsvorrichtung 14 erfasst wird, und andererseits den gespeicherten Volumenströmen für das irrigationsfluid und das Aspirationsfluid vorgenommen. Die erfassten Werte und die gespeicherten Werte werden zueinander korreliert, woraufhin die Okklusionsbestimmungsvorrichtung 15 einen Okklusionszustand bestimmen kann.

Von der Okklusions-Bestimmungsvorrichtung 15 wird ein Okklusionszustands-Signal S1 an ihrem Ausgang bereitgestellt, welches einem Eingang einer Steuerungseinrichtung 16 zugeführt wird. Die Steuerungseinrichtung 16 verarbeitet dieses Okklusionszustands-Signal S1 und gibt an ihrem Ausgang ein Signal S2 als Steuersignal für mindestens einen Betriebsparameter des ophthalmochirurgischen Handstücks 6 an das ophthalmochirurgische Handstück 6 ab. Der Betriebsparameter kann eine elektrische Energie sein. Ein Handstück für die Phakoemulsifikation weist üblicherweise Piezokeramiken als Stellglieder auf, an welche eine höhere oder niedrigere Spannung angelegt werden kann. Damit ändert sich die Auslenkung der Piezokeramiken und damit der Stellweg oder Hub der Hohlnadel des Handstückes. Wenn das Vorliegen einer Okklusion von der Okklusions-Bestimmungsvorrichtung erkannt wird, kann das Handstück mit einer höheren Energie versorgt werden, sodass ein die Hohlnadel verstopfendes Partikel zerkleinert werden kann. Dies gilt in umgekehrter Weise genauso: Wenn das Aufbrechen einer Okklusion erkannt wird, kann weniger oder gar keine Energie an das Handstück zugeführt werden, da kein Partikel mehr die Hohlnadel verstopft.

Gemäß der Ausführungsform in Fig. 1 wird zusätzlich das Okklusionszustands-Signal S1 einem ersten Zeitglied 21 und einem zweiten Zeitglied 22 zugeführt. Das erste Zeitglied 21 ist eingerichtet, in Abhängigkeit von dem Okklusionszustands-Signal S1 einen ersten Irrigationsfluid-Solldruck in Abhängigkeit von der Zeit zu ermitteln und ein zugehöriges Signal S3 für die erste Fluidpumpe 5 bereitzustellen. Wenn zum Beispiel von der Okklusions-Bestimmungsvorrichtung 15 erkannt wird, dass eine vollständige Okklusion an der Hohlnadel des ophthalmochirurgischen Handstückes 6 vorliegt, kann der Irrigationsfluid-Solldruck während eines vorbestimmten Zeitraumes abgesenkt werden. Damit wird verhindert, dass dem Auge zusätzliches Irrigationsfluid zuströmt und den Augeninnendruck erhöht. Gleichzeitig kann mit dem zweiten Zeitglied 22 ein Aspirationsfluid-Solldruck in Abhängigkeit von der Zeit ermittelt und ein zugehöriges Signal S4 für die zweite Fluidpumpe 11 bereitgestellt werden. Bei einer vollständigen Okklusion kann dies bedeuten, dass mittels der zweiten Fluidpumpe 11 vergleichbar mit einer Pulsfunktion sofort ein deutlich höherer Unterdruck in der Aspirationsfluidleitung 10 angelegt wird, um das Partikel an der Hohlnadel-Spitze zu halten und zu zerbrechen.

Bei der in Fig. 1 dargestellten Ausführungsform werden das Signal S3 und S4 nicht direkt der ersten Fluidpumpe 5 bzw. der zweiten Fluidpumpe 11 zugeführt. Vielmehr wird das Signal S3 einem ersten Eingang einer ersten Auswertevorrichtung 31 zugeführt, welches an einem zweiten Eingang ein Signal S5 von einem dritten Zeitglied 33 empfängt. Das dritte Zeitglied 33 wiederum ist mit einer Fußbedienungseinheit in Form eines Fußpedals 35 gekoppelt, welches von einem Bediener betätigt werden kann. Analog dazu wird das Signal S4 einem ersten Eingang einer zweiten Auswertevorrichtung 32 zugeführt, welches an einem zweiten Eingang ein Signal S6 von einem vierten Zeitglied 33 empfängt. Das vierte Zeitglied 34 ist mit dem Fußpedal 35 gekoppelt. Ein Bediener kann damit vorgeben, wie schnell die Signale S3 und S4 zu einer Änderung des Irrigationsfluid-Solldrucks und Aspirationsfluid-Solldrucks führen sollen, woraufhin an einem Ausgang der ersten Auswertevorrichtung 31 ein zugehöriges Signal für den Irrigationsfluid-Steuerdruck an die erste Fluidpumpe 5 und an einem Ausgang der zweiten Auswertevorrichtung 32 ein zugehöriges Signal für den Aspirationsfluid-Steuerdruck der zweiten Fluidpumpe 11 zugeführt wird.

Von Bedeutung ist, dass durch die erfindungsgemäße ophthalmochirurgische Einrichtung nur dann Energie einem ophthalmochirurgischen Handstück 6 zugeführt wird, wenn eine Okklusion erkannt wird. Durch den Vergleich von gespeicherten zeitlichen Verläufen eines Irrigationsfluid-Volumenstroms und Aspirationsfluid-Volumenstroms mit einem momentan erfassten zeitlichen Verlauf von Irrigationsfluid und Aspirationsfluid ist es möglich, nur eine minimal erforderliche Energie zuzuführen. Die Gefahr einer Überhitzung zum Beispiel im Bereich der Einstichstelle der Hohlnadel an der Hornhaut und damit eine Verbrennung der Hornhaut wird dadurch deutlich verringert. Die erfindungsgemäße ophthalmochirurgische Einrichtung reduziert damit die Gefahr von medizinischen Komplikationen und erhöht die Sicherheit eines ophthalmochirurgischen Eingriffs.

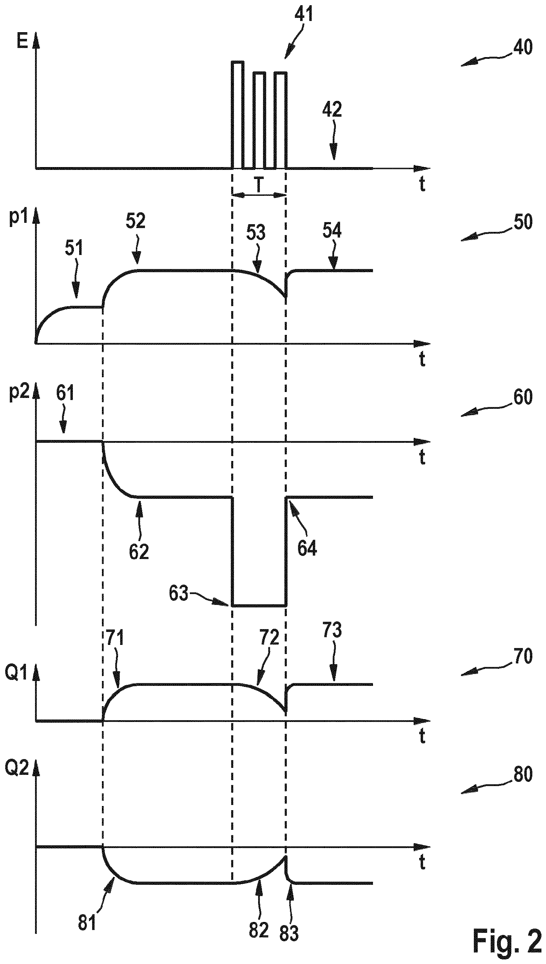

In Fig. 2 sind einige Diagramme von Betriebsgrößen in Abhängigkeit von der Zeit t dargestellt. Das Diagramm 40 zeigt einen Verlauf von Energiepulsen, das Diagramm 50 zeigt einen Verlauf eines Irrigationsfluiddruckes p1 in einer Irrigationsfluidleitung, Diagramm 60 zeigt einen Verlauf eines Aspirationsfluiddruckes p2 in einer Aspirationsfluidleitung, Diagramm 70 zeigt einen Verlauf eines ersten Volumenstroms Q1 in einer Irrigationsfluidleitung, und Diagramm 80 zeigt einen Verlauf eines zweiten Volumenstroms Q2 in einer Aspirationsfluidleitung.

Nachfolgend werden die Kurvenverläufe jeweils mit Bezugszeichen erläutert. Wenn zu Beginn ein Irrigationsfluid in eine Irrigationsfluidleitung strömt, liegt in der Irrigationsfluidleitung ein erster Druck vor, siehe Bezugszeichen 51. In diesem Moment liegt noch kein Unterdruck in der Aspirationsfluidleitung vor, siehe 61. Wenn dann die erste Fluidpumpe 5 das Irrigationsfluid fördert, steigt in der Irrigationsfluidleitung der erste Volumenstrom Q1 an, siehe 71. Gleichzeitig wird die zweite Fluidpumpe 11 in Betrieb gesetzt, sodass in der Aspirationsfluidleitung der zweite Volumenstrom Q2 ansteigt, siehe 81. In der Irrigationsfluidleitung steigt der Irrigationsfluiddruck an, siehe 52, und in der Aspirationsfluidleitung steigt der Unterdruck an, siehe 62. Wenn keine Okklusion auftritt, verbleiben die Werte für den Irrigationsfluiddruck, den Aspirationsfluiddruck, den ersten Volumenstrom und den zweiten Volumenstrom auf ihrem Niveau. Wenn aber eine Okklusion beginnt, verringert sich in der Aspirationsleitung der zweite Volumenstrom, siehe 82, und in der Irrigationsfluidleitung verringert sich der erste Volumenstrom, siehe 72. Gut erkennbar ist, dass diese Verringerung der Volumenströme symmetrisch zueinander verläuft. Mittels der Okklusionsbestimmungs-Vorrichtung 15 wird dies erkannt, woraufhin die zweite Fluidpumpe 11 angesteuert wird, einen relativ hohen Unterdruck in der Aspirationsleitung zu bewirken, siehe 63. Damit die zweite Fluidpumpe 11 so schnell reagieren kann, ist sie bevorzugt eine Membranpumpe. Dies gilt auch für die erste Fluidpumpe 5, welche bevorzugt eine Membranpumpe ist. Die Membranpumpe kann wie in DE 102016201297 B3 beschrieben aufgebaut sein.

Die Okklusionsbestimmungs-Vorrichtung 15 ist auch mit der Steuerungseinrichtung 16 gekoppelt. Die Steuerungseinrichtung 16 gibt ein Steuersignal für einen Betriebsparameter wie zum Beispiel die elektrische Energie an das ophthalmochirurgische Handstück 6. In diesem Beispiel wird ein Handstück für die Phakoemulsifikation angenommen, wobei das Handstück mit Energiepulsen angesteuert wird, siehe 41. Die Energiepulse können mit zunehmender Zeitdauer abnehmen, konstant bleiben oder steigen, sodass die Darstellung in Fig. 2 nur ein Beispiel darstellt. Es kann auch das Verhältnis zwischen Pulsdauer und Pulspause verändert werden (Duty Cycle), und bei einem Vitrektom kann die Schneidrate verändert werden. Zusätzlich wird die erste Fluidpumpe 5 so angesteuert, dass der Druck p1 in der Irrigationsfluidleitung abnimmt, siehe 53.

In dem Beispiel sei angenommen, dass aufgrund der angelegten Energiepulse 41 und dem hohen Unterdruck p2 in der Aspirationsfluidleitung das Partikel an der Spitze der Hohlnadel 7 des Handstücks 6 in kleinere Teile zerbricht und die Okklusion damit endet. Bei dem noch hohen Unterdruck in der Aspirationsfluidleitung nimmt der zweite Volumenstrom schnell wieder zu, siehe 83, und auch der erste Volumenstrom in der Irrigationsleitung nimmt schnell wieder zu, siehe 73. Die wird von der Okklusionsbestimmungs-Vorrichtung 15 erkannt. Daraufhin wird die zweite Fluidpumpe 11 so angesteuert, dass der hohe Unterdruck sofort reduziert wird, siehe 64. Gleichzeitig werden die Energiepulse sofort beendet, siehe 42. Zusätzlich wird die erste Fluidpumpe 5 so angesteuert, dass wieder ein zuvor angelegter Druck in der Irrigationsleitung erreicht wird, siehe 54.

Es wird darauf hingewiesen, dass nur der erste Volumenstrom Q1 und der zweite Volumenstrom Q2 bestimmt werden. Der jeweilige Druck p1 in der Irrigationsleitung 4 und Druck p2 in der Aspirationsleitung 10 werden nicht bestimmt. Die in den Diagrammen 50 und 60 gezeigten Druckverläufe sind nur zur Erläuterung dargestellt, werden aber nicht mit der ophthalmochirurgischen Einrichtung gemessen.

Wenn das ophthalmochirurgische Handstück ein Vitrektom ist, kann der Betriebsparameter für das Vitrektom die elektrische Energie für eine Schneidrate sein. Die Energie kann also dazu genutzt werden, dass das Schneidelement mit höherer Frequenz betrieben wird. In dem Diagramm 40 kann dann statt Energiepulsen in dem Zeitraum T die Schneidrate deutlich höher sein als in den Zeiträumen davor und danach.

### Bezugszeichen:

- 1: Ophthalmochirurgische Einrichtung
- 2: Irrigationsfluidbehälter
- 3: Irrigationsfluid
- 4: Irrigationsfluidleitung
- 5: erste Fluidpumpe
- 6: Ophthalmochirurgisches Handstück
- 7: Hohlnadel
- 8: Auge
- 10: Aspirationsfluidleitung
- 11: zweite Fluidpumpe
- 12: Sammelbehälter
- 13: erste Volumenstrom-Bestimmungsvorrichtung
- 14: zweite Volumenstrom-Bestimmungsvorrichtung
- 15: Okklusionsbestimmungs-Vorrichtung
- 16: Steuerungseinrichtung
- 21: erstes Zeitglied
- 22: zweites Zeitglied
- 31: erste Auswertevorrichtung
- 32: zweite Auswertevorrichtung
- 33: drittes Zeitglied
- 34: viertes Zeitglied
- 35: Fußpedal
- 40, 50, 60, 70, 80: Diagramme
- 41, 42, 51-54, 61-: Betriebszustände in den Diagrammen 40, 50, 60, 70, 80
- 64, 71-73, 81-83 p1: Irrigationsfluiddruck
- p2: Aspirationsfluiddruck
- Q1: erster Volumenstrom
- Q2: zweiter Volumenstrom
- S1: Okklusionszustands-Signal
- S2: Steuersignal für Handstück
- S3: Signal für ersten Irrigationsfluid-Solldruck
- S4: Signal für ersten Aspirationsfluid-Solldruck
- S5: Signal für zweiten Irrigationsfluid-Solldruck
- S6: Signal für zweiten Aspirationsfluid-Solldruck
- T: Zeitraum der Okklusion

## Patentansprüche

1. Ophthalmochirurgische Einrichtung (1), aufweisend:
- Eine Irrigationsfluidleitung (4), durch welche Irrigationsfluid (3) von einem Irrigationsfluidbehälter (2) zu einem ophthalmochirurgischen Handstück (6) strömen kann,
- eine Aspirationsfluidleitung (10), durch welche Aspirationsfluid von dem ophthalmochirurgischen Handstück (6) zu einem Sammelbehälter (12) strömen kann,
- eine erste Volumenstrom-Bestimmungsvorrichtung (13), welche in Strömungsrichtung vor dem Handstück (6) angeordnet ist und eingerichtet ist, einen ersten Volumenstrom (Q1) in der Irrigationsfluidleitung (10) zu bestimmen,
- eine zweite Volumenstrom-Bestimmungsvorrichtung (14), welche in Strömungsrichtung nach dem Handstück (6) angeordnet ist und eingerichtet ist, einen zweiten Volumenstrom (Q2) in der Aspirationsfluidleitung (10) zu bestimmen,
**gekennzeichnet durch**
- eine Okklusions-Bestimmungsvorrichtung (15), welche mit der ersten Volumenstrom-Bestimmungsvorrichtung (13) und der zweiten Volumenstrom-Bestimmungsvorrichtung (14) gekoppelt ist und eingerichtet ist, den zeitlichen Verlauf des ersten Volumenstroms (Q1) und den zeitlichen Verlauf des zweiten Volumenstroms (Q2) zu erfassen, jeweils einen Vergleich mit gespeicherten zeitlichen Verläufen eines ersten Volumenstroms in der Irrigationsfluidleitung bei unterschiedlichen Okklusionszuständen und gespeicherten zeitlichen Verläufen eines zweiten Volumenstroms in der Aspirationsfluidleitung bei unterschiedlichen Okklusionszuständen vorzunehmen und auf Basis der Vergleiche einen momentanen Okklusionszustand in der Aspirationsfluidleitung (10) zu bestimmen.

2. Ophthalmochirurgische Einrichtung (1) nach Anspruch 1, welche eine Steuerungseinrichtung (16) aufweist, welche eingerichtet ist, an einem Eingang von der Okklusions-Bestimmungsvorrichtung (15) ein Okklusionszustands-Signal (S1) zu empfangen und an einem Ausgang ein Steuersignal (S2) für mindestens einen Betriebsparameter des ophthalmochirurgischen Handstücks (6) an das ophthalmochirurgische Handstück (6) abzugeben.

3. Ophthalmochirurgische Einrichtung (1) nach Anspruch 2, wobei der Betriebsparameter des ophthalmochirurgischen Handstücks (6) eine elektrische Antriebsenergie ist.

4. Ophthalmochirurgische Einrichtung (1) nach Anspruch 3, wobei die elektrische Antriebsenergie für eine longitudinale Bewegung oder transversale Bewegung oder Torsions-Bewegung einer Piezokeramik eines ophthalmochirurgischen Handstückes (6) zur Phakoemulsifikation vorgesehen ist.

5. Ophthalmochirurgische Einrichtung (1) nach Anspruch 3, wobei die elektrische Antriebsenergie für eine longitudinale Bewegung oder transversale Bewegung oder Torsions-Bewegung oder rotatorische Bewegung eines Schneidelementes eines Vitrektoms vorgesehen ist.

6. Ophthalmochirurgische Einrichtung (1) nach einem der vorherigen Ansprüche, aufweisend:
- eine erste Fluidpumpe (5), welche in Strömungsrichtung zwischen dem Irrigationsfluidbehälter (2) und der ersten Volumenstrom-Bestimmungsvorrichtung (13) angeordnet ist und eingerichtet ist, Irrigationsfluid (3) zum Handstück (6) zu fördern,
- eine zweite Fluidpumpe (11), welche in Strömungsrichtung zwischen der zweiten Volumenstrom-Bestimmungsvorrichtung (14) und dem Sammelbehälter (12) angeordnet ist und eingerichtet ist, Aspirationsfluid zum Sammelbehälter (12) zu fördern,
- ein erstes Zeitglied (21), welches eingerichtet ist, an seinem Eingang das Okklusionszustands-Signal (S1) zu empfangen, in Abhängigkeit von dem Okklusionszustands-Signal (S1) einen ersten Irrigationsfluid-Solldruck in Abhängigkeit von der Zeit zu ermitteln und an seinem Ausgang ein Signal (S3) für den ersten Irrigationsfluid-Solldruck der ersten Fluidpumpe (5) abzugeben,
- ein zweites Zeitglied (22), welches eingerichtet ist, an seinem Eingang das Okklusionszustands-Signal (S1) zu empfangen, in Abhängigkeit von dem Okklusionszustands-Signal (S1) einen ersten Aspirationsfluid-Solldruck in Abhängigkeit von der Zeit zu ermitteln und an seinem Ausgang ein Signal (S4) für den ersten Aspirationsfluid-Solldruck der zweiten Fluidpumpe (11) abzugeben.

7. Ophthalmochirurgische Einrichtung (1) nach Anspruch 6, aufweisend:
- eine erste Auswertevorrichtung (31), welche eingerichtet ist, an einem ersten Eingang das Signal (S3) für den ersten Irrigationsfluid-Solldruck zu empfangen, und an einem zweiten Eingang ein Signal (S5) für einen zweiten Irrigationsfluid-Solldruck zu empfangen, und an einem Ausgang ein Signal für einen Irrigationsfluid-Steuerdruck der ersten Fluidpumpe (5) zuzuführen, und
- eine zweite Auswertevorrichtung (32), welche eingerichtet ist, an einem ersten Eingang das Signal (S4) für den ersten Aspirationsfluid-Solldruck zu empfangen, und an einem zweiten Eingang ein Signal (S6) für einen zweiten Aspirationsfluid-Solldruck zu empfangen, und an einem Ausgang ein Signal für einen Aspirationsfluid-Steuerdruck der zweiten Fluidpumpe (11) zuzuführen.

## Claims

1. Ophthalmic surgical device (1), comprising:
- an irrigation fluid line (4) through which irrigation fluid (3) can flow from an irrigation fluid container (2) to an ophthalmic surgical handpiece (6),
- an aspiration fluid line (10) through which aspiration fluid can flow from the ophthalmic surgical handpiece (6) to a collecting container (12),
- a first volumetric flow rate determination apparatus (13) which is arranged upstream of the handpiece (6) in the flow direction and which is configured to determine a first volumetric flow rate (Q1) in the irrigation fluid line (10),
- a second volumetric flow rate determination apparatus (14) which is arranged downstream of the handpiece (6) in the flow direction and which is configured to determine a second volumetric flow rate (Q2) in the aspiration fluid line (10),
**characterized by**
- an occlusion determination apparatus (15) which is coupled to the first volumetric flow rate determination apparatus (13) and to the second volumetric flow rate determination apparatus (14) and which is configured to acquire the time curve of the first volumetric flow rate (Q1) and the time curve of the second volumetric flow rate (Q2), to respectively carry out a comparison with stored time curves of a first volumetric flow rate in the irrigation fluid line for different occlusion states and stored time curves of a second volumetric flow rate in the aspiration fluid line for different occlusion states, and to determine a current occlusion state in the aspiration fluid line (10) on the basis of the comparisons.

2. Ophthalmic surgical device (1) according to Claim 1, comprising a control device (16) which is configured to receive an occlusion state signal (S1) from the occlusion determination apparatus (15) at an input and to output a control signal (S2) for at least one operational parameter of the ophthalmic surgical handpiece (6) to the ophthalmic surgical handpiece (6) at an output.

3. Ophthalmic surgical device (1) according to Claim 2, wherein the operational parameter of the ophthalmic surgical handpiece (6) is an electrical drive power.

4. Ophthalmic surgical device (1) according to Claim 3, wherein the electrical drive power is provided for a longitudinal movement or transverse movement or torsional movement of a piezoceramic of an ophthalmic surgical handpiece (6) for phacoemulsification.

5. Ophthalmic surgical device (1) according to Claim 3, wherein the electrical drive power is provided for a longitudinal movement or transverse movement or torsional movement or rotational movement of a cutting element of a vitrectome.

6. Ophthalmic surgical device (1) according to any of the preceding claims, comprising:
- a first fluid pump (5) which is arranged between the irrigation fluid container (2) and the first volumetric flow rate determination apparatus (13) in the flow direction and which is configured to convey irrigation fluid (3) to the handpiece (6),
- a second fluid pump (11) which is arranged between the second volumetric flow rate determination apparatus (14) and the collecting container (12) in the flow direction and which is configured to convey aspiration fluid to the collecting container (12),
- a first timing element (21) which is configured to receive the occlusion state signal (S1) at its input, to ascertain as a function of time a first irrigation fluid target pressure on the basis of the occlusion state signal (S1), and to output a signal (S3) for the first irrigation fluid target pressure of the first fluid pump (5) at its output,
- a second timing element (22) which is configured to receive the occlusion state signal (S1) at its input, to ascertain as a function of time a first aspiration fluid target pressure on the basis of the occlusion state signal (S1), and to output a signal (S4) for the first aspiration fluid target pressure of the second fluid pump (11) at its output.

7. Ophthalmic surgical device (1) according to Claim 6, comprising:
- a first evaluation apparatus (31) which is configured to receive the signal (S3) for the first irrigation fluid target pressure at a first input and to receive a signal (S5) for a second irrigation fluid target pressure at a second input, and to supply a signal for an irrigation fluid control pressure of the first fluid pump (5) at an output, and
- a second evaluation apparatus (32) which is configured to receive the signal (S4) for the first aspiration fluid target pressure at a first input and to receive a signal (S6) for a second aspiration fluid target pressure at a second input, and to supply a signal for an aspiration fluid control pressure of the second fluid pump (11) at an output.

## Revendications

1. Instrument chirurgical ophtalmique (1), comprenant :
- une conduite (4) de fluide d'irrigation, à travers laquelle un fluide d'irrigation (3) peut s'écouler d'un récipient (2) de fluide d'irrigation vers une pièce (6) à main chirurgicale ophtalmique,
- une conduite (10) de fluide d'aspiration, à travers laquelle un fluide d'aspiration peut s'écouler de la pièce (6) à main chirurgicale ophtalmique vers un récipient (12) de collecte,
- un premier dispositif (13) de détermination de débit volumétrique, qui est disposé en amont de la pièce (6) à main dans la direction d'écoulement et est conçu pour déterminer un premier débit volumétrique (Q1) dans la conduite (10) de fluide d'irrigation,
- un second dispositif (14) de détermination de débit volumétrique, qui est disposé en aval de la pièce (6) à main dans la direction d'écoulement et est conçu pour déterminer un second débit volumétrique (Q2) dans la conduite (10) de fluide d'aspiration,
**caractérisé par**
- un dispositif (15) de détermination d'occlusion, qui est couplé au premier dispositif (13) de détermination de débit volumétrique et au second dispositif (14) de détermination de débit volumétrique et est conçu pour détecter l'évolution temporelle du premier débit volumétrique (Q1) et l'évolution temporelle du second débit volumétrique (Q2), pour effectuer respectivement une comparaison avec des évolutions temporelles mémorisées d'un premier débit volumétrique dans la conduite de fluide d'irrigation dans différents états d'occlusion et des évolutions temporelles mémorisées d'un second débit volumétrique dans la conduite de fluide d'aspiration dans différents états d'occlusion et, sur la base des comparaisons, pour déterminer un état d'occlusion instantané dans la conduite de fluide d'aspiration.

2. Instrument chirurgical ophtalmique (1) selon la revendication 1 comprenant un dispositif (16) de commande, qui est configuré pour recevoir, sur une entrée, un signal d'état d'occlusion (S1) en provenance du dispositif (15) de détermination d'occlusion et pour délivrer, sur une sortie, à la pièce (6) à main chirurgicale ophtalmique, un signal de commande (S2) correspondant à au moins un paramètre de fonctionnement de la pièce (6) à main chirurgicale ophtalmique.

3. Instrument chirurgical ophtalmique (1) selon la revendication 2, dans lequel le paramètre de fonctionnement de la pièce (6) à main chirurgicale ophtalmique est une énergie d'entraînement électrique.

4. Instrument chirurgical ophtalmique (1) selon la revendication 3, dans lequel l'énergie d'entraînement électrique est prévue pour un mouvement longitudinal ou un mouvement transversal ou un mouvement de torsion d'une piézocéramique d'une pièce (6) à main chirurgicale ophtalmique servant à la phacoémulsification.

5. Instrument chirurgical ophtalmique (1) selon la revendication 3, dans lequel l'énergie d'entraînement électrique est prévue pour un mouvement longitudinal ou un mouvement transversal ou un mouvement de torsion ou un mouvement de rotation d'un élément de coupe d'un instrument de vitrectomie.

6. Instrument chirurgical ophtalmique (1) selon l'une quelconque des revendications précédentes, comprenant :
- une première pompe (5) à fluide, qui est disposée dans la direction d'écoulement entre le récipient (2) de fluide d'irrigation et le dispositif de détermination de débit volumétrique (13), et est conçue pour acheminer le fluide d'irrigation (3) vers la pièce (6) à main,
- une seconde pompe (11) à fluide, qui est disposée dans la direction d'écoulement entre le dispositif (14) de détermination de débit volumétrique et le récipient (12) de collecte, et est conçue pour acheminer le fluide d'aspiration vers le récipient (12) de collecte,
- un premier temporisateur (21), qui est conçu pour recevoir sur son entrée le signal d'état d'occlusion (S1), pour déterminer, en fonction du signal d'état d'occlusion (S1), une première pression de consigne du fluide d'irrigation en fonction du temps, et pour délivrer sur sa sortie un signal (S3) correspondant à la première pression de consigne du fluide d'irrigation de la première pompe (5) à fluide,
- un second temporisateur (22), qui est conçu pour recevoir sur son entrée le signal d'état d'occlusion (S1), pour déterminer, en fonction du signal d'état d'occlusion (S1), une première pression de consigne du fluide d'aspiration en fonction du temps, et pour délivrer sur sa sortie un signal (S4) correspondant à la première pression de consigne du fluide d'aspiration de la seconde pompe (11) à fluide.

7. Instrument chirurgical ophtalmique (1) selon la revendication 6, comprenant :
un premier dispositif (31) d'évaluation, qui est conçu pour recevoir, sur une première entrée, le signal (S3) correspondant à la première pression de consigne du fluide d'irrigation et pour recevoir, sur une seconde entrée, un signal (S5) correspondant à une seconde pression de consigne du fluide d'irrigation, et pour délivrer, sur une sortie, un signal correspondant à une pression de commande du fluide d'irrigation de la première pompe (5) à fluide, et un second dispositif (32) d'évaluation, qui est conçu pour recevoir, sur une première entrée, le signal (S4) correspondant à la première pression de consigne du fluide d'aspiration et pour recevoir, sur une seconde entrée, un signal (S6) correspondant à une seconde pression de consigne du fluide d'aspiration, et pour délivrer, sur une sortie, un signal correspondant à une pression de commande du fluide d'aspiration de la seconde pompe (11) à fluide.
